# EUROPÄISCHE PATENTANMELDUNG

(11) **EP 1 637 149 A1**
(43) Veröffentlichungstag der Anmeldung: **22.03.2006**
(21) Anmeldenummer: 04013556.8
(22) Anmeldetag: 09.06.2004
(51) Int. Cl.: A61K 36/00, A61K 9/48, A61P 13/10

(54) **Nahrungsergänzungsmittel für den Mann**

(71) Anmelder: Medifood GmbH, 50825 Köln (DE)
(72) Erfinder: Flach, Alexander, 50933 Köln (DE)
(74) Vertreter: Helbing, Jörg

(57) **Zusammenfassung**

Die Erfindung betrifft eine als Nahrungsergänzungsmittel bzw. Arzneimittel geeignete Zusammensetzung, die eine polyphenolhaltige Substanz pflanzlichen Ursprungs (insbesondere Traubenkernextrakt (*Vitis vinifera*)), eine ΨΨ-Carotinverbindung (insbesondere Lycopin) und wenigstens eine potenzstärkende/prosexuelle Substanz pflanzlichen Ursprungs umfasst. Die Zusammensetzung ist insbesondere zur Prävention von männerspezifischen Alterungserscheinungen, insbesondere sexueller Dysfunktion, Herz-Kreislauf Problemen, Fettstoffwechselerkrankungen und Blasenschwäche geeignet.

## Beschreibung

Die Erfindung betrifft eine als Nahrungsergänzungsmittel bzw. Arzneimittel geeignete Zusammensetzung, die eine polyphenolhaltige Substanz pflanzlichen Ursprungs (insbesondere Traubenkernextrakt *(Vitis vinifera)), e*ine ψψ-Carotinverbindung (insbesondere Lycopin) und wenigstens eine potenzstärkende/prosexuelle Substanz pflanzlichen Ursprungs umfasst. Die Zusammensetzung ist insbesondere zur Prävention von männerspezifischen Alterungserscheinungen, insbesondere sexueller Dysfunktion, Herz-Kreislauf Problemen, Fettstoffwechselerkrankungen und Blasenschwäche geeignet.

### Hintergrund der Erfindung

Männer haben andere Gesundheitsrisiken als Frauen, sie sind allgemein in einem schlechteren Gesundheitszustand und haben eine kürzerer Lebenserwartung als Frauen (S.J. Loeb, Geriatric Nursing 24(5): 278-85 (2003)). Diese Problematik wird besonders offensichtlich mit zunehmenden Alter. Ein Ansatz ist daher das Ansprechen einer neuen Zielgruppe, die "jungen-alten" Männer (40+ Jahre), wie es von Williams *et al.* (Journal of Health Education 29:166-173 (1996)) gefordert wird. Präventive Maßnahmen, die einfach in das Leben zu integrieren sind, haben dabei die besten Erfolgsaussichten. Ein ganzheitlicher, holistischer Ansatz ist dabei vorzuziehen, so dass Männergesundheit als ein ganzheitliches Kontinuum angegangen werden sollte.

Der Begriff des "Aging Male" ist Teil der Lifestyle Medizin geworden, und umfasst nicht nur die Sexualfunktion des Mannes, sondern auch allgemeine Leistungsschwäche und Abgeschlagenheit wie sie einhergehen kann mit erniedrigtem Testosteronspiegel (Tsujimura A. et al., Journal of Urology 170(6 Pt 1) :2345-7 (2003))g Dieses Mangelsyndrom ist auch bekannt unter dem Namen partielles Androgen-Defizit Syndrom (PADAM) und ist unter anderem gekennzeichnet durch reduzierte Libido und Potenz. Des weiteren stellen Prostataleiden, wie die Prostatavergrößerung (benignes Prostatasyndrom), Prostataentzündung (Prostatitis) und das Prostatakarzinom, sowie Blasenentzündung (Zystitien) gesundheitliche Probleme des "Aging Male" dar. Störungen des Fettstoffwechsels, die sich in einem zu hohen Gehalt an Blutfetten äußern (Hyperlipidämie) treten ebenfalls verstärkt ab dem vierzigsten Lebensjahr auf (Lesho EP et al., American Family Physician 69(3): 525-32 (2004)). Erhöhte Blutfette, insbesondere Erhöhungen des Cholesterins, sind eine Hauptursache bei der Entstehung der Arterienverkalkung (Artheriosklerose), wodurch das Risiko für weitere Herz-Kreislauf-Erkrankungen (vaskuläre/koronare Erkrankungen) erheblich zunimmt. Symptome die auf eine vaskulären Erkrankung hindeuten sind unter anderem das Auftreten erektiler Dysfunktionen (Kloner R.A., Current Urology Reports 4(6):466-71 (2003)). Studien aus den USA haben gezeigt, dass ein Mann aus zehn an erektiler Dysfunktion leidet, und andere Studien sprechen von 52 % der Männer im Alter von 40 bis 70 die an verschieden gradigen Formen der Impotenz leiden (Fine, S.R., The Journal of the American Osteopathic Association 104(1 Suppl. 1): 9-15 (2004)).

Bevor eine medizinische Intervention nötig wird, kann der einzelne Mann, allein durch eine bewusste Ernährung, die über die ausgewogene Ernährung hinausgeht, viel für seine eigene Gesundheit tun. Dem steht aber häufig eine geschäftige Lebensweise entgegen, die geprägt ist von Fastfood und Fertiggerichten. Es kommt zur Nährstoffunterversorgung, wobei besonders die Mikronährstoffaufnahme betroffen ist, die nur durch gezielte Nährstoffaufnahme auszugleichen ist. Klinische Mangelsymptome stehen in der Regel erst am Ende einer längeren Phase mit niedriger Mikronährstoffaufnahme (Vitamine, Mineralien und weitere Bestandteile wie Carotinoide, Flavonoide, Anthozyane etc.). Eine inadäquate Versorgung mit Mikronährstoffen auf molekularer Ebene kann Veränderungen verursachen, die die Grundlage für die Entwicklung von chronischen Erkrankungen und beschleunigten Alterungsprozessen sind und die sich erst später manifestieren.

Das eine Verbindung besteht zwischen der Ernährung, Langlebigkeit und dem Auftreten von Krebs und Herz-Kreislauferkrankungen ist seit langem bekannt (Leis H.P. Jr., International Surgery 76(1): 1-5 (1991); Suzuki et al. Asia Pacific Journal of Clinical Nutrition 10(2):165-71 (2001)). Antioxidanzien scheinen von besonderer Wichtigkeit zu sein. Antioxidanzien schützen vor freien Sauerstoffradikalen (Singulett Sauerstoff), die auf molekularer Ebene oxidativen Stress verursachen. Daher gelten Antioxidanzien auch als wichtiger Schutz vor der Entstehung verschiedener Krebsarten. Die antioxidativ wirksamsten Vitamine sind die Vitamine C, E und die ψψ -Carotine. Das ß-Carotin (ββ-Carotin) dahingegen ist ein sogenanntes Provitamin, da es ein Vorläufer von Vitamin A ist.Der sekundäre Pflanzenstoff Lycopin ist ein Carotinoid aus der Gruppe der ψψ-Carotine, das keinen geschlossenen β-Iononring aufweist. Es kommt als orange-rotes Hauptcarotinoid in hohen Konzentrationen (ca. 3 mg/100 g) in Tomaten *(Lycopersicon lycopersicum*) vor. In vergleichbaren Konzentrationen ist Lycopin in roter/rosé Grapefruit, Guaven und Wassermelonen enthalten. Auch Hagebutten, die Früchte der Hundsrose *(Rosa canina)* haben einen sehr hohen Gehalt an Lycopin. Die Gehalte liegen bei ca. 3-5 mg / 100 g. L. besitzt keine Provitamin-A-Aktivität, wirkt jedoch als Antioxidans. Im Vergleich zu β-Carotin führen die geöffneten Iononringe im Molekül zu einer effektiveren Quenchrate von Singulettsauerstoff. Im Tierversuch wirkt Lycopin anticancerogen und daher ist dieser sekundäre Pflanzenstoff von Bedeutung für die menschliche Ernährung. Lycopin kann in Zellkultur unter Laborbedingungen das Wachstum von Prostatakrebszellen hemmen. Aus epidemiologischen Daten lässt sich entnehmen, dass die Höhe der Lycopinaufnahme mit dem Risiko an Prostatakrebs zu erkranken, invers korreliert (Lu Q. Y. *et al.,* Cancer Epidemiology Biomarkers and Prävention 10(7): 749-56 (2001)). Diese drei Vitamine wirken synergistisch. Neben diesen Vitaminen haben auch Spurenelementen wie Selen und Zink antioxidative Wirkung und werden für die Prostatakrebsprophylaxe empfohlen.

Flavonoide (Polyphenole), insbesondere Procyanidin, wirken als weitaus stärkere Antioxidanzien als die oben beschriebenen Vitamine und aus Tiermodellen ist ihre anticarcinogene Wirkung bekannt (Zhao J. *et al,* Carcinogenesis 20(9): 1737-1745 (1999). Procyanidin ist als Antioxydans 20x stärker als Vitamin C und 50x stärker als Vitamin E (Shi J. *et al.,* Journal Medicinal Food 6(4):291-9 (2003)). Bislang gibt es aber keine generelle Empfehlungen zum Einsatz dieser Antioxidans für männerspezifische Anwendungen. Procyanidine finden sich in hoher Konzentration in Traubenkernen (Yilmaz Y. and Toledo R. T., Journal of Agricultural and Food Chemistry 52(2): 255-60 (2004)). Die gezielte Verwendung von Traubenkernextrakt erhöht die totale antioxidative Aktivität des Blutserum (Nuttall S.L. *et al.,* Journal of Clinical Pharmacy and Therapeutics 23(5): 323-325 (1998)). Weitere Quellen für Polyphenole sind Apfelkerne, Grapefruitkerne und Rotwein.

Bei der Bekämpfung von Herz-Kreislauf Erkrankungen spielt ein Vitamin B, die Folsäure, eine gewichtige Rolle. Eine Studie mit 46000 Männern hat gezeigt, dass die Gabe von Folsäure das Risiko an einer Herz-Kreislauf Erkrankung zu erliegen, enorm verringert (Merchant *et al.,* Journal of Nutrition 133(9): 2863-2867 (2003)).

Die Senkung des Blut Lipidspiegels wirkt sich ebenfalls positiv auf das Herz-Kreislauf-System auszuwirken. Pflanzliche Extrakte haben eine lange Tradition bei der Senkung des Lipidspiegels (Thompson Coon J.S. and Ernst E., Journal of Family Practice 52(6): 468-478 (2003)). Extrakte aus Artischocken *(Cyanara scolymus),* Bockshornklee *(Trigonella foenum-graecum)* und das Harz des Guggul Baums *(Commiphora mukul)* können bekanntlich den Gesamtcholesterinspiegel zwischen 10 und 33 % senken. In der Naturheilkunde werden auch Löwenzahn *(Taraxacum officicinale*), Mariendistel *(Carduus marianus)* und Schwarzkümmelöl *(Nigella sativa)* zur Senkung des Lipidspiegels eingesetzt. Das Schwarzkümmelöl ist ein Synergist zu Artischockenextrakt. Dieses Öl enthält über 80 % mehrfach ungesättigte Fettsäuren, die für zahlreiche Lebensfunktion eine bedeutende Rolle spielen, insbesondere für die Zellatmung. Sie sind weiterhin beteiligt an der Synthese von Prostaglandinen, die auf den Ablauf vieler Körperfunktionen, wie Gehirnleistung, Nervenleitung, Freisetzung von Neurotransmittern, Senkung des Blutdrucks, Aktivierung des Immunsystems und der Hormonsekretion allgemein regulierend einwirken. Schwarzkümmel enthält 0,5-1 % an etherisches Öl (Inhaltsstoffe Nigellin und Melanthin), welches neben der antioxidativen Wirkung, auch antibakteriell und antimykotisch wirkt. Nigellin und Melanthin wirken weiterhin sekretlösend, reinigend und austreibend (Ratgeber Gesundheit, Sweetnews, biozac.de)

Bei dem benignen Prostatasyndrom (Symptomatik: häufiges Wasserlassen, plötzlich zwingender Harndrang, nächtliches Wasserlassen, Restharngefühl, abgeschwächter Harnstrahl, Startverzögerung) finden auch pflanzliche Drogen Anwendung. Aus der Volksmedizin ist seit langem bekannt, dass Brennnesselwurzel-Extrakte *(Urtica dioica)* bei Prostataleiden helfen können.

Wirkstoffe wie das beta-Sitosterol, Scopoletin und Urtica-Agglutinine senken das Sexualhormon bindende Globulin SHGB und hemmen die Aromataseaktivität und damit die Wirkung männlicher und weiblicher Geschlechtshormone auf das Prostatawachstum. Kürbissamen und daraus gewonnene Extrakte *(Curcubita pepo)* zeichnen sich durch das Vorkommen von Curcubitin und Phytosterolen in freier und glycosidisch gebundener Form aus. Einige der Phytosterole hemmen die Dihydrotestosteronsynthese und beeinflussen die Prostaglandinsynthese. Weiteres Wachstum der Prostata wird dadurch gehemmt. Extrakte aus Sägepalmenfrüchten *(Serine repens, Sabal serulata)* hemmen die 5-Alphareduktase und vermindern damit die Synthese von Dihydrotestosteron, welches die benigne Prostatahyperplasie stimuliert. Extrakte aus Sägepalmenfrüchten hemmen ebenfalls die Aromatase und haben spasmolytische und antiproliferative Wirkung auf die Prostata. Roggenpollenextrakt *(Secale cereale*) enthält Lipoxygenasen und andere Wirkstoffe die zum einen spasmolytisch wirken, zum anderen die Prostatazellvermehrung hemmen. In der Wurzel der südafrikanischen Lilie *(Hypoxis rooperi)* wurden hohe Anteile an beta-Sitosterplen gefunden, für die eine hemmende Wirkung auf die Leukotrien- und Prostaglandinsynthese angenommen wird, die sich antiphlogistisch und tonusstabilisierend positiv auf Prostatabeschwerden auswirken.

Zur Stärkung der Blasenfunktion findet in der Naturheilkunde seit langem die Selleriewurzel Anwendung. Die Selleriewurzel wirkt ausleitend, entgiftend und auch kräftigend. Sellerie ist ein Stärkungsmittel. Die stärkende Wirkung geht auf den Inhaltsstoff Apiol zurück. Sellerie regt den Appetit an und stärkt die allgemeine Nieren- und Drüsentätigkeit. Durch die Aufnahme von Sellerieextrakt kommt es auch zu einer psychischen Stärkung, da Sellerieextrakt nervöse Unruhe vorbeugt und so hilft psychischen Stress abzubauen (Monographie Kommission E: Datei-Nr.: 49029, Fachforum Kamal Sabri Kolta: Altägyptische Heilpflanzen - einst und heute, Institut für Geschichte der Medizin der LMU, München, Naturheilpraxis 1/2001). Weiterhin werden Preiselbeerextrakte *(Vaccinium vitis-idaea),* Extrakte und Saft der großfrüchtigen Moosbeere *(Vaccinium macrocarpon),* Wiesenküchenschellextrakt *(Pulsatilla pratensis)* bei wiederkehrenden Blasenentzündungen eingesetzt.

Seit Alters her bekannt sind auch pflanzliche potenzstärkende Mittel und Aphrodisiaka, die die Libido und die sexuelle Potenz wiederherstellen, steigern, oder, dem männlichen Alterungsprozess zuwider, lange erhalten sollen. Eines der bekanntesten aphrodisischen Pflanzen ist der sibirische Ginseng *(Eleutherococcus senticosus maxim (Acanthopanax senticosus))* wie auch der Ginseng *(Panax ginseng).* Die im Ginseng enthaltenen Ginsenoside führen zur steigern die NO Synthese in verschiedenen Organen, einschließlich in den Schwellkörpern *(Corpora cavernosa).* Weiterhin verstärken sie die Acetylcholin-induzierte und durch transmurale Nervenstimulierung ausgelöste Entspannung, die einhergeht mit der Ausschüttung von cyklischen Guanosinmonophosphat (Murphy L. L. and Lee T. J., Annals of the New York Academy of Sciences 962:372-7 (2002); Sandroni P, Clinical Autonomic Research 11(5):303-7 (2001)). Darauf beruht die potenzstärkende Wirkung von Ginseng.

Beliebt bei Indianerstämmen Mittel- und Südamerikas sind Damiana (*Turneara diffusa),* Extrakte. Sie gehörte zu den wichtigsten Heilkräutern der Maya und Azteken, und gelangte im 17. Jahrhundert nach Europa, wo der Name "Hemdauszieher" wegen ihrer stark aphrodisischen Wirkung geprägt wurde. Neuere Forschung an impotenten Ratten hat deren Wirksamkeit belegt (Arletti R. *et al.,* Psychopharmacology 143(1): 15-19 (1999). Eine Zwillingsstudie an Männern die ein Damiana enthaltenes Nahrungsergänzungsmittel (ArginMax) erhielten, beobachtete eine Verbesserung der Erektionsfähigkeit (Ito *et al.,* Hawaii Medical Journal 57: 741-744 (1998).

Ein weiteres aus der Volksmedizin Südamerikas stammendes potenzstärkendes Mittel ist die Maca Wurzel *(Maca andina; Lepidium meyenii).* Auch hier haben neuere Studien an Ratten deren Wirksamkeit belegt (Cicero A.F. *et al,* Journal of Ethnopharmacology 75(2-3): 225-229 (2001); Cicero A.F. *et al,* Andrologia 34(3): 177-179 (2002)). Menschliche Studien fehlen jedoch.

Ein wenig untersuchtes pflanzliches potenzstärkendes Mittel ist der aus der Rinde des Catuababaums gewonnene Tee. Ihm wird eine belebende, krampflösende Wirkung nachgesagt, und über einen längeren Zeitraum getrunken verbessert er die Durchblutung des ganzen Körpers, wodurch auch das Libido und Potenz gestärkt werden sollen. Ebenfalls aus der Volksmedizin stammt der Glaube an die potenzstärkende Wirkung des gemahlenen Holzes des Sassafrasbaum. Als "leichte Liebesdroge" wird das Sassafrasbaumholz vertrieben, ohne das dazu aber fundierte wissenschaftliche Studien dies belegen. Die Anwendung erscheint jedoch bedenklich, da das ätherische Öl von Sassafras Safrol enthält, das bekanntermaßen eine kanzerogene, leber- und nervenschädigende Wirkung aufweist, und dessen Einnahme zu Übelkeit, Erbrechen, Schock und zentralnervösen Störungen führt. Selbst kosmetische Produkte mit Sassafras, die geringe Mengen an Safrol enthalten können, dürfen nicht bei Kindern angewendet werden.

Extrakte aus der Cola-Nuss *(Cola nitida)* finden ebenfalls Verwendung in potenzstärkenden Mitteln. Die Wirkung der Cola-Nuss ist unspezifisch stimulierend, aufgrund ihres hohen Coffein Gehalts (Morton J.F., Basic Life Scieneces 59:739-65 (1992)).

Extrakte aus der Rinde des Yohimbe-Baums *(Pausinystalia yohimbe),* insbesondere das Alkaloid-Yohimbin werden seit langem als potenzstärkendes Mittel verwendet. Die Wirkung des Yohimbin wird mit einer erhöhten Reflexerregbarkeit im sakralen Rückenmark, verursacht durch die Hemmung der alpha₂-adrenergenen Rezeptoren und mit einer gesteigerten Blutzufuhr in die Penisschwellkörper *(Corpora cavernosa und Corpus spongiosum)* erklärt. Mehrere Studien haben die Wirksamkeit bei leichten bis mittelgradigen organischen Impotenzursachen belegt (Morales A., International Journal of Impotence Research 12 (suppl.1): 70-74 (2000); Morales *et al.* Journal of Urology 137: 1168-1172 (1998); Rowland *et al.,* Archives of Sexual Behaviour 26: 49-62 (1997); Carey, M.P. and Johnson, B.T., Archives of Sexual Behaviour 25: 341-360 (1996)).

Der Rinde und dem Holz des Muira-puama-Baums *(Ptychopetalum olacoides Benth.; Ptychopetalum Anselmino)* wird ebenfalls potenzstärkende Wirkung zugesprochen. Beide Pflanzenbestandteile gehören seit Jahrhunderten zu den indianischen Arzneien. Das harte Baumholz ist auch unter dem Namen Potenzholz bekannt, denn das geraspelte Holz und die Innenrinde wurden zur Steigerung des Lustempfindens und der Potenz verwendet. Verschiedene Studien belegen eine allgemeine aphrodisische Wirkung, wie auch positive Effekte auf erektile Dysfunktionen des Muira-puama-Holz, insbesondere auch bei älteren Männern, die unter Müdigkeit leiden und von allgemeinen Alterungserscheinungen betroffen sind (Sandoval, A. Natural Health 30: 40; Waynberg, J. American Journal of Natural Medicine 1: 8-9 (1994).

Weiterhin ist Haferstroh *(Avena sativa)* ein traditionelles allgemeines Stärkungsmittel, dem auch potenzstärkende Wirkung nachgesagt wird. Nachgewiesenermaßen ist Haferstroh reich an Antioxidanzien (Handelman *et al.,* Journal of Agricultural and Food Chemistry 47(12): 4888-4893 (1999)), die sich positiv auf den allgemeinen Gesundheitszustand eines Mannes auswirken.

An Naturheilverfahren, pflanzliche Arzneien und Nahrungsergänzungsmittel besteht ein großes Interesse, und dem Interessierten steht ein großes Angebot offen. Verschiedenen Zusammensetzungen werden auf dem Markt angeboten. Oftmals handelt es sich um Produkte die nur einen aktiven Inhaltsstoff haben, wie beispielsweise Artischockenextrakt (Artischocke Madaus mit Artischockenblättern-Trockenextrakt, (Madaus AG, Köln)), oder solche die sich nur auf ein gesundheitliches Problem gerichtet sind. Beispiele für solche Produkte sind solche, die sich alleine gegen Verdauungsprobleme richten (Lipei® mit 400 mg Trockenextrakt aus Artischockenblättern (4-6:1) (Steiner Arzneimittel Berlin) ), oder solche die alleine gegen Störungen des Fettstoffwechsels wirken (Cynara Aar® mit 150 mg Trockenextrakt aus frischen Artischockenblättern (25-35:1); Artischockendragees (Aar Pharma GmbH & Co.KG, Remscheid), z.B. Zirkulin mit 46 % Artischockenextrakt (Zirkulin Naturheilmittel GmbH, Bremen), Gastro Intakt (Queisser Pharma, Flensburg), Galle-Dragee mit 300 mg Trockenextrakt aus Artischockenblättern (5,8-7,5:1) (Pro-Dimi Pharma, Dortmund), Artischockenkaspeln z.B. von: Astrid-Twardy GmbH; Pharma Peter; Aalborg Pharma; Diamant Natur B.V.; Biolog. Praep. Dr. Gross), ). Sehr verbreitet sind auch Vitaminpräparate alleine oder in Kombination mit Mineralstoffen (z.B. Zentrum). Produkte, die speziell an männliche Konsumenten herangetragen werden, sind solche, die sich gegen Beschwerden des Prostata/Blasenkomplex richten, z.B. das apothekenpflichtige "Prostagutt® ", das Extrakte der Sägepalmenfrucht und der Brennnessel enthält, das apothekenpflichtige "GranuFink® ", das eine Wirkstoffkombination aus Kürbis und Sägepalmenfrucht enthält, aber auch apothekenpflichtige Einstoffpräparate wie das "Azuprostat® Urtica", das Brennnesselextrakt enthält. Entsprechende nicht-apothekenpflichtige Zusammensetzungen werden auch im Internet und in Reformhäusern angeboten. Weitere Produkte die sich speziell an männlichen Konsumenten wenden, sind solche die Verbesserung der sexuellen Leistungsfähigkeit versprechen. Diese enthalten Muira-puama-Holz *(Ptychopetalum olacoides Benth.; Ptychopetalum Anseimino),* Yohimbe-Rinde *(Corynanthe yohimbe),* Damiana *(Turnera diffusa),* Maca-Wurzel *(Maca andina; Lepidium meyenii),* Sibirischen Ginseng (Eleutherococcus senticosus maxim (Acanthopanax senticosus)), Catuaba-Rinde *(Erythroxylon catuaba),* Sassafrasholz (Sassafras albidum), Cola-Nuss *(Cola nitida),* Haferstroh *(Avena sativa)* etc. alleine oder in Kombination. Beispiele für solche Produkte, wie sie im Internet Angeboten werden, sind "Male Plus" (Amazon Formula for Men), welches nachfolgende Stoffe enthält: Muira puama, Catuaba, Sarsasparilla und Damiana; "Yohimbe Plus™", dass lediglich Yohimbe enthält; "Phytagra", mit Auszügen aus Muira puama, Schizandra, Siberischen Ginseng, Ginkgo biloba. Dann gibt es auch solche Produkte, die neben den potenzstärkenden pflanzlichen Extrakten auch Mineralstoffe enthalten; z.B. Super "MiraForte", welches Zink, Chrysin (ein Isoflavon aus der Passionsblume ebenfalls mit potenzstärkender Wirkung), Muira puama, Maca, Brennnessel, Ingwer und Bioperine® Piperine (Extrakt aus schwarzen Pfeffer) enthält. Die sogenannte "Circulation Formula" verspricht eine verbesserte Zirkulation einhergehend mit sexueller Leistungssteigerung, wobei dieses Produkt die Aminosäure Arginin, B-Vitamine, Muira-puama-Extrakt, Ginkgo biloba Extrakt, Rosskastanienextrakt und Mäusedornwurzelstockextrakt *(Rusci aculeati rhizoma)* enthält.

Neben diesen auf dem Markt befindlichen Produkten, wurden auch verschiedene Zusammensetzungen in Patentanmeldungen bzw. Patenten offenbart:
FR 2710267 beschreibt eine Zusammensetzung zur Behandlung von Impotenz, umfassend 6 bis 11.5 % Ursodeoxychol Säure, 20 bis 40 % an Panax Ginseng Extrakt, 2.5 bis 6 % an Muira puama Extrakt, 0.7 bis 1.5 % of Yohimbine Hydrochlorid, 3 bis 6.5 % of Guarana Extrakt, 0.2 bis 0.6 % an *Nux vomica* (Brechnuss) Extrakt, 6.5 % bis 12 % an Damiana Extrakt, 8 bis 14 % Nicotinamid, 6 bis 11.5 % an Nicotinsäure, % bis 6.5 % L-Tocopherolacetat, 0.003 bis 0.01 % Clenbuterolhydrochlorid und 12 bis 20 % eines Trägerstoffs. Diese Zusammensetzung lässt gänzlich außer Acht, dass Potenzproblem oftmals nur eine Symptom für Herz-Kreislauf Erkrankungen sind, und diese auch therapiert werden sollten, um eine optimale sexuelle Leistungssteigerung zu erzielen. Weiterhin enthält es ein Anabolikum Clenbuterolhydrochlorid, was in ganz Europa verschreibungspflichtig ist, da es unerwünschte Nebenwirkungen verursacht.

In DE 4007975 wird ein Aphrodisiakum auf der Basis pflanzlicher und/oder tierischer Substanzen offenbart. Bei den tierischen Substanzen handelt es sich um Testes siccatum (pulverisiertes Hodengewebe) und Cantharis und bei den pflanzlichen Substanzen um aus Holz, Blättern und/oder Wurzeln extrahierte Wirkstoffe von Muira puama, Damianae sowie Ginseng. Auch diese Zusammensetzung verfolgt keinen ganzheitlichen Ansatz der Gesundheit eines Mannes, sondern ist nur auf Potenzprobleme gerichtet, und lässt viele andere Gesundheitsaspekte außer Acht. Weiterhin enthält das Aphrodisiakum tierische Substanzen und ist somit ungeeignet für Vegetarier.

Das US Patent US 6,444,237 beschreibt eine Zusammensetzung, zur positiven Beeinflussung der sexuelle Dysfunktion. An aktiven Substanzen enthält diese Zusammensetzung L-Arginin, L-Theanin, L-Glutamat, *Crataegus monogyna* Beeren-extrakt, *Turnera diffusa* Extrakt, *Pfaffia paniculata* Extract, *Ginkgo biloba* Extrakt, *Pygeum africanum* Extrakt, and Ginsenoside. Diese Zusammensetzung ist alleine auf Wiederherstellung der sexuellen Funktionen gerichtet.

In der US 2002/0068728 werden Zusammensetzungen zur Steigerung der Libido offenbart. Die für Männer vorgeschlagene Zusammensetzung umfasst *Tribulus terrestris, Muria puama, Catuba amazonas* Rinde, Androstenedion, L-Arginin, koreanischen Ginseng, *Avena sativa,* und natürliches Vitamin E.

Die südafrikanische Patentanmeldung ZA 98/8241 beschreibt eine Zusammensetzung zur Behandlung der erektilen Dysfunktion und/oder des Libidoverlusts. Wesentliche Bestandteile sind Muira puama, L-Arginin und L-Histidin; optionale Bestandteile sind Zink Glukonat, Vitamin B1, Vitamin B2, Vitamin B6, Vitamin B12, Nicotin Säure (Vitamin B3), Vitamin E, sibirischer Ginseng, Folsäure, Calcium Pantothenat (Vitamin B5) und Eisenlactat.

WO 02/051426 offenbart eine Zusammensetzung zur Behandlung der sexuellen Dysfunktion, zur Verstärkung der sexuellen Leistungsfähigkeit, zur Anhebung des Testosteron Spiegels und zur Verbesserung der Muskelkraft und Muskelmasse. Wesentliche Bestandteile dieser Zusammensetzung sind Yohimbe und ein Vorläufer von Testosteron, z.B. 4-Androstenedion. Daneben kann diese Zusammensetzung auch noch weitere Extrakte z.B. von *Epimedium saggitatum, Tribulus terrestris, Ptychopetalum olacoides* Wurzel (Muira puama), und *Serenoa repens* (Sägepalmenfrüchten) enthalten. Auch diese Anmeldung enthält keine Hinweise, wie andere männliche Gesundheitsprobleme angegangen werden können.

DE 3931693 offenbart ein Sexualtonikum für Männer. Dieses ist eine Zusammensetzung, umfassend alkoholische Extrakte aus Hafer *(Avena sativa),* Safran *(Crocus sativus),* Feige *(Ficus carica*) und Schilfwurzeln *(Phragmites communis).*

Die EP1344529 offenbart eine Zusammensetzung zur Behandlung der männlichen Glatzenbildung, die in einem Pflaster zu verabreicht ist. Die Zusammensetzung umfasst die Antioxidans Chrysin und/oder Muira puama, *Tribulas terrestris,* Brennesselextrakt, *Eurycoma longifolia jack* und/oder *Avena sativa.* Die in dieser Anmeldung offenbarte Zusammensetzung dient lediglich einem einzigen männlichen Gesundheitsaspekt, und ignoriert alle übrigen männlichen Gesundheitsprobleme.

WO 0124805 offenbart eine Zusammensetzung zur Erniedrigung des Lipidspiegels. Diese Zusammensetzung enthält wenigstens eine pflanzliche Substanz ausgewählt aus der nachstehenden Gruppe: *Anethum graveolens, Citrus limonum Risso, Cynara Scolymus* and *Cichorium intybus,* sowie eine Substanz ausgewählt aus Vitamin C, Vitamin E und einem ß-Carotin.

BG 106355, US 6,197309 und US 6,241,987 offenbaren Zusammensetzungen die bei Prostata und Blasenbeschwerden hilfreich sind. Wesentliche Bestandteile sind in BG 106355 das Öl der Sägepalmenfrucht und Kürbiskernöl. Wesentliche Bestandteile sind in US 6,197309 Vitamin C, Vitamin B6, Vitamin E, Zink, Glycin, L-Alanin, Glutamat, Sägepalmenfrucht, Pygeum Extrakt, Kürbiskerne, Brennnessel, Echinacea, Knoblauch, Ginkgo Blätter und Selen. Wesentliche Bestandteile sind in US 6,241,987 Sägepalmenfruchtextrakt, Kürbiskernextrakt und Brennnesselextrakt.

Neben den alleine auf sexuellen Funktionswiederherstellung gerichtete Patente/Patentanmeldungen, offenbaren andere Zusammensetzungen, die anderen Aspekten der Gesundheit dienlich sind.

So offenbart WO 01/15552 ein Nahrungsergänzungsmittel zur Senkung der Serum Triglyzeride und des Cholesterins. Entscheidende Einzelbestandteile sind eine Omega-3 Fettsäure und ein Phytosterol, wobei bevorzugte Quelle für die Omega-3 Fettsäure Fischöl ist. Auch diese Zusammensetzung hat lediglich einen gesundheitlichen Aspekt ins Auge gefasst und ist da sie tierische Substanzen enthält ungeeignet für Vegetarier.

FR 2739530 offenbart eine Zusammensetzung zur Entgiftung/Entschlackung, die unter anderem Sellerie (Apium graveolens) und Artischocke *(Cyanara scolymus)* umfasst.

WO 03075861 offenbart eine antioxidative Zusammensetzung, enthaltend (a) mindestens zwei Extrakte ausgewählt aus *Vaccinium myrtillus, Trifolium pratense, Vitis vinifera* sowie *Thea vinensis,* und (b) Vitamin E und/oder Vitamin C. Diese Zusammensetzung dient als "Anti-Ageing" -Mittel.

Der bisherige Stand der Technik bietet bislang keine Zusammensetzung, die allen wesentlichen Aspekten der Gesundheit eines Mannes dient. Bisherige Zusammensetzungen fokussieren sich lediglich auf ein männliches Gesundheitsproblem, lassen aber andere Aspekte außer Acht. Diese begrenzte Vorgehensweise ist suboptimal, da sie den Mann als Ganzes ignoriert.

### Zusammenfassung der Erfindung

Überraschenderweise wurde gefunden, dass eine rein pflanzliche Zusammensetzung, die zwingend eine polyphenolhaltige pflanzliche Substanz, eine ψψ-Carotinverbindung und wenigstens eine potenzstärkende und/oder eine prosexuelle pflanzliche Substanz enthält, sich positiv auf alle wesentlichen männlichen Befindlichkeitsstörungen und das Wohlbefinden (und den Gesundheitszustand) von Männern, insbesondere von Männern über 40 Jahre auswirkt. In dieser Altersgruppe manifestieren sich vermehrt erste Alterungserscheinungen wie Probleme mit Potenz, Prostata, Blase, Fettstoffwechsel und dem Herz-Kreislauf-System, ohne jedoch so schwerwiegend zu sein, dass medizinische Intervention gesucht wird. Für diese Probleme erweist sich die erfindungsgemäße Zusammensetzung als optimal, da sie mittels pflanzlicher Extrakte, die allgemein gut verträglich sind, sehr effektiv diesen organischen Problemen entgegenwirkt, bzw. bereits existierende organische Probleme positiv beeinflusst. Die Zusammensetzung ist sehr effektiv, da sich Synergieeffekte aus den besonders ausgewählten Einzelkomponenten ergeben. Dadurch erzielt die Zusammensetzung eine enorm verbesserte Wirkung im Vergleich zu Einstoffpräparaten, oder Zusammensetzungen mit anderen Bestandteilen.

Die Erfindung betrifft
(1) Eine Zusammensetzung umfassend a) eine polyphenolhaltige Substanz pflanzlichen Ursprungs (b) eine ψψ-Carotinverbindung und (c) wenigstens eine potenzstärkende und/oder prosexuelle Substanz pflanzlichen Ursprungs;
(2) eine bevorzugte Ausführungsform von (1), wobei die Zusammensetzung als polyphenolhaltige Substanz Traubenkernextrakt *(Vitis vinifera),* als ψψ-Carotinverbindung Lycopin, und als potenzstärkende Substanz Muira-puama-Holz Extrakt oder als prosexuelle Substanz Haferstrohextrakt enthält;
(3) eine bevorzugte Ausführungsformen von (1) und (2), umfassend weiterhin
   - eine pflanzliche Substanz, die den Fettstoffwechsel-stimuliert, insbesondere einen Extrakt aus Artischockenblättern; und/oder
   - eine pflanzliche Substanz mit antikongestiver, antiproliferativer, antiphlogistischer und/oder antiandrogener Wirkung auf das benigne Prostatasyndrom, insbesondere Kürbiskernextrakt; und/oder
   - einen pflanzlichen Extrakt mit harntreibender Wirkung, insbesondere Sellerieextrakt; und/oder
   - Mineralstoffe und/oder Spurenelemente und/oder Vitamine;
(4) eine bevorzugte Ausführungsform von (3), wobei die Bestandteile der Zusammensetzung in nachfolgende Gewichtsprozenten vorliegen:
   Muira puama Extrakt: 0,8 - 2,7 Gew.-%
   Traubenkernextrakt: 4,9 - 14,8 Gew.-%;
   Lycopin:0,8 - 2,7 Gew.-%;
   Haferstroh Extrakt: 14 - 44 Gew.-%;
   Artischockenblätter Extrakt: 3,9 - 11,8 Gew.-%;
   Kürbiskern Extrakt: 13 - 42 Gew.-%;
   Selleriewurzel Extrakt: 2,7 - 8,2 Gew.-%; und ein Spurenelement
   Vitamin-Premix enthaltend 10 - 30 µg Selen, 1 - 5 mg Zink, 100 - 300 µg Folsäure, 33 - 100 µg Biotin, 30 - 100 mg Vitamin C, 3,7 - 14 mg Vitamin E, 0.5 -1,5 mg Vitamin B6 und 1 - 3 mg β-Carotin;
(5) Ein Verfahren zur Herstellung der Zusammensetzung (1) bis (4) umfassend das Vermischen der Komponenten (a) bis (h); und
(6) Eine Kapsel enthaltend eine der Zusammensetzungen gemäß den Ausführungsformen (1) bis (4).

Die erfindungsgemäße Zusammensetzung verwendet nur hochreine Pflanzenextrakte. Sie ist zur Prävention von männerspezifischen Alterungserscheinungen, insbesondere sexueller Dysfunktion, Herz-Kreislauf Problemen, Fettstoffwechselerkrankungen und Blasenschwäche geeignet.

### Detaillierte Beschreibung der Erfindung

Der erfindungsgemäße Gegenstand ist eine Zusammensetzung, die als Nahrungsergänzungsmittel für Männer eingesetzt wird. Diese Zusammensetzung ist auf die Bedürfnisse von Männern, insbesondere von Männern über 40 Jahren zugeschnitten. Besonderheit dieser Zusammensetzung ist, dass sie dem Zellschutz dienende Substanzen enthält, die synergistisch erste Alterungserscheinungen eines Mannes, wie Probleme mit Potenz, Prostata, Blase, Fettstoffwechsel und dem Herz-Kreislauf-System bekämpfen. Als Konsequenz stellt sich ein höheres Wohlbefinden und ein allgemein besserer Gesundheitszustand beim Mann ein. Eine Besonderheit der erfindungsgemäßen Zusammensetzung ist, dass die aktiven Bestandteile in geringer Dosierung vorliegen, durch die Synergieeffekte, die Zusammensetzung aber wirksamer ist als vergleichbare Einstoffpräparate, mit höherer Dosierung, und auch wirksamer als andere Mehrstoffpräparate mit anderen Einzelkomponenten.

"Nahrungsergänzungsmittel" im Sinne der vorliegenden Erfindung sind Lebensmittel, die dazu bestimmt sind, die normale Ernährung zu ergänzen. Sie bestehen aus Einfach- oder Mehrfachkonzentraten von Nährstoffen oder sonstigen Stoffen mit ernährungsspezifischer oder physiologischer Wirkung und werden in dosierter Form in den Verkehr gebracht, d. h. z.B. in Kapsel, Pastillen, Tabletten, Pillen, Dragees, Pulverbeuteln, oder entsprechenden Darreichungsformen, als Flüssigkeiten in Flüssigampullen, Flaschen mit Tropfeinsätzen, oder ähnlichen Darreichungsformen, und als Pulver, zur Aufnahme von abgemessenen kleinen Mengen (Richtlinie 2002/46/EG v. 10.06.2002, L 183/51-57).

Eine "Substanz" im Sinne der vorliegenden Erfindung ist ein pflanzlicher Wirkstoff, der zu Arzneizwecken und Nahrungsergänzungsmittelzwecken verwendet werden kann. Eine solche Substanz pflanzlichen Ursprungs oder eine Zubereitung aus einer solchen wird insgesamt als ein wirksamer Bestandteil betrachtet, unabhängig davon, ob die wirksamkeitsbestimmenden, d. h. therapeutisch aktiven Inhaltsstoffe, bekannt sind. Die Substanzen umfassen dabei Extrakte aber auch Öle und Pulver der Pflanzen.

"Extrakte" im Sinne der vorliegenden Erfindung umfassen Fluid-, Spissum- und Trockenextrakte. Native (lat.: *nativus,* angeboren, natürlich, unverändert) Extrakte werden aus frischen (z.B. frisches Blattwerk *(herba)* einer Pflanzendroge) und natürlichen Ausgangsstoffen gewonnen. Native Extrakte sind immer Vielkomponentengemische, wobei neben dem Hauptwirkstoff noch Begleitstoffe vorliegen, die die Wirkung des Hauptwirkstoffs unterstützen, indem sie beispielsweise dessen Resorption oder auch dessen Stabilität erhöhen, und somit als Co-Effektoren wirken.

Ein "Antioxidanz" im Sinne der vorliegenden Erfindung ist eine Verbindung, die als "Radikalfänger" wirkt, d. h. freie Sauerstoffradikale (Singulett Sauerstoff) inaktiviert. Sauerstoffradikale sind hochreaktiv, und führen zu Oxidationsvorgängen an biologischen Molekülen, ein Vorgang der als oxidativer Stress bezeichnet wird.

Ein "Stärkungsmittel" im Sinne der vorliegenden Erfindung ist ein Mittel mit Wirkstoffen, die den körperlichen und psychischen Allgemeinzustand verbessern. Synonyme sind Kräftigungsmittel, Roborans und Aufbaupräparat. Sie entfalten ihre stärkende Wirkung dadurch, dass sie allgemein appetitanregend wirken und so Erschöpfungszuständen entgegenwirken (Meyers Enzyklopädisches Lexikon. Bd. 21, S. 561-562 (1980), Brockhaus Gesundheit, 6, Aufl. Mannheim F.A. 2003).
"Herz-Kreislauf-Erkrankungen" im Sinne der vorliegenden Erfindung bezeichnen alle Störungen des Herz-Kreislauf-Systems, die mit Artherioskterose und Vorstufen dieser in Verbindung stehen.

"Potenzstärkende Wirkung" im Sinne der vorliegenden Erfindung umfasst sowohl die Wiederherstellung, Steigerung und das Erhalten der Libido wie auch der sexuellen Potenz (erektile Funktion).

"Kapsel" im Sinne der vorliegenden Erfindung bezeichnet eine feste Zusammensetzung umgeben von einer harten oder einer weichen Hülle von unterschiedlicher Form oder Größe, zur peroralen Einnahme. Es zählen hierzu Hartgelatinekapseln, Weichgelatinekapseln, Hydroxypropylmethylcellulose (HPMC) Kapseln, magensaftresistente Kapseln, Kapseln mit modifizierter Wirkstofffreisetzung. Die erfindungsgemäße Kapsel (6) enthält die erfindungsgemäße Zusammensetzung, und optional Zusatzstoffe z.B. solche, die der Zerfallsbeschleunigung der Pulvermischung (der Zusammensetzung) dienen, wie Füllstoffe, die der Verbesserung der Verdichtungseigenschaften dienen, Fließregulierungsmittel, die der Verbesserung des Fließverhaltens und Reduktion der Adhäsion an Metallflächen dienen, Benetzungsmittel, die der Verbesserung der Wasserpenetration dienen, Farbstoffe, die der Unterscheidung der Kapsel sowie der Beeinflussung der Psyche dienen usw.

Die Zusammensetzung (1) der Erfindung umfasst eine polyphenolhaltige Substanz (insbesondere Traubenkernextrakt *(Vitis vinifera)),* eine ψψ-Carotinverbindung, insbesondere Lycopin, und eine potenzstärkende und/oder eine prosexuelle Substanz pflanzlichen Ursprungs.

Traubenkernextrakt ist reich an Phytochemikalien, insbesondere Flavonoide, sowohl in monomerer Form als auch in komplexer Form. In der Tat stellen Traubenkerne und durch Kaltpressung gewonnene Extrakte und Mehle die reichste Quelle für das komplexe Flavonoid Procyanidin dar. Procyanidin ist die stärkste natürliche Antioxidans und ist 20x stärker als Vitamin C und 50x stärker als Vitamin E. Procyanidin wirkt als "Superprotektor" und unterstützt und ergänzt andere Vitamine in deren Wirkung. Procyanidin, und das Procyanidin enthaltene Traubenkernextrakt verbessert auch die Blutzirkulation und fördert die Versorgung der Gehirnzellen mit Sauerstoff und Nährstoffen. Eine bevorzugte Ausführungsform der erfindungsgemäßen Zusammensetzung zeichnet sich durch ihren Anteil nativen Traubenkernextrakt, welches besonders reich an der Antioxidans Procyanidin ist, aus.

Daneben enthält die erfindungsgemäße Zusammensetzung als ψψ-Carotinverbindung vorzugsweise Lycopin. Dieses ist aus einer speziellen Tomatensorte, die besonders reich an Lycopin ist, durch Sprühtrocknung erhältlich. Alternative Quellen für Lycopin sind Hagebutten, die Früchte der Hundsrose *(Rosa canina*), roter/rosé Grapefruit, Guaven und Wassermelonen. Lycopin ist ebenfalls eine Anitioxidans, die sich besonders durch ihre präventive Wirkung auf Prostatakrebs auszeichnet. Die Kombination von Traubenkernextrakt, reich an Procyanidinen und Lycopin bewirkt einen bislang unbeobachteten Synerergieeffekt im Blutserum, so dass die messbare antioxidative Aktivität im Blutserum höher ist, als bei alleiniger Verwendung von nur Traubenkernextrakt oder nur Lycopin. Hieraus ergeben sich die positiven Effekte auf den allgemeinen Gesundheitszustand des Mannes, insbesondere für die Gesundheit der Prostata, wie auch des Herz-Kreislauf-Systems.
In einer bevorzugten Ausführungsform enthält die erfindungsgemäße Zusammensetzung weiterhin ein potenzstärkendes Extrakt oder eine potenzstärkende pflanzliche Substanz. Diese sind vorzugsweise ausgewählt aus Muira-puama-Holz *(Ptychopetalum olacoides Benth.; Ptychopetalum Anseimino),* Yohimbe-Rinde *(Corynanthe yohimbe),* Damiana *(Turnera diffusa),* Maca-Wurzel *(Maca andina; Lepidium meyenii),* Sibirischer Ginseng (Eleutherococcus senticosus maxim (Acanthopanax senticosus)), Catuaba-Rinde *(Erythroxylon catuaba),* und Cola-Nuss *(Coala vera).* Besonders bevorzugt ist eine erfindungsgemäße Zusammensetzung, in der das potenzstärkendes Extrakt oder die potenzstärkende pflanzliche Substanz Muira-puama-Holz *(Ptychopetalum olacoides Benth.; Ptychopetalum Anselmino)* oder *Muira-puama-(Ptychopetalum olacoides)-Rinde* ist. Das Muira-puama-Holz Extrakt fördert besonders die Potenz/Männlichkeit, vitalisiert und verstärkt den Sexualtrieb. In Kombination mit den anderen beiden Bestandteilen der Zusammensetzung hat es eine vitalisierende Wirkung auf die Sexualfunktion des Mannes. Diese Wirkung kann noch verstärkt werden durch die Beifügung eines weiteren prosexuellen pflanzliches Extrakt, insbesondere von Haferstroh *(Avena sativa)* Extrakt, oder auch eines der anderen oben aufgelisteten potenzstärkenden Extrakten oder potenzstärkenden pflanzlichen Substanzen. Haferstroh wirkt als ein allgemeines Stärkungsmittel.

Eine Zusammensetzung aus Traubenkernextrakt *(Vitis vinifera),* Lycopin, Extrakten aus Muira puama *(Ptychopetalum olacoides Benth.; Ptychopetalum Anselmino*), und Haferstroh *(Avena sativa)* hat sich dabei als besonders wirkungsvoll erwiesen, aufgrund von unerwarteten Synergieeffekten.

Weiterhin wurde gefunden, dass durch Zusatz wenigstens eines den Fettstoffwechsel-stimulierenden pflanzlichen Extrakts eine Zusammensetzung mit noch höherem Gesundheitswert geschaffen wurde. Diese pflanzlichen Extrakte wirken sich positiv auf den Leberstoffwechsel und den Gallenstoffwechsel aus. Sie regen eine verstärkte Gallensekretbildung und dessen Ausschüttung an. Die Fettemulgierung und damit die Verdauung der Nahrungsfette erfolgt dadurch effektiver, was sich insgesamt Triglycerid- und Cholesterinreduzierend im Blutserum auswirkt. Die den Fettstoffwechsel-stimulierende pflanzliche Extrakt können ausgewählt werden aus der Gruppe von nachfolgenden Extrakten und/oder Ölen: Artischocke *(Cyanara cardunculus),* Löwenzahn *(Taraxacum officinale),* Mariendistel *(Carduus marianus),* Schwarzkümmel, Bockshornklee *(Trigonella foenum-graecum)* und das Harz des Guggul Baums *(Commiphora mukul).* Besonders bevorzugt ist eine Zusammensetzung die Traubenkernextrakt *(Vitis vinifera),* Lycopin, Extrakte aus Muira-puama-Holz(Ptychopetalum *olacoides Benth.; Ptychopetalum Anseimino),* Haferstroh *(Avena sativa)* und Artischocke *(Cyanara cardunculus),* umfasst. Diese Kombination eignet sich zur allgemeinen Gesunderhaltung des Mannes, indem es den Fettstoffwechsel optimiert und dadurch unter anderem das Vorkommen von dyspeptischen Beschwerden verringert. Diese Zusammensetzung hat sich auch als sehr effektiv erwiesen in der Verringerung von Herz-Kreislauf-Beschwerden, die verbunden sind mit einem zu hohen Lipidspiegel im Blutserum.

Eine Zusammensetzung mit positiver Wirkung auf das benigne Prostatasyndrom ist erhältlich durch Zusatz von wenigstens einem pflanzlichen Extrakt mit antikongestiver, antiproliferativer, antiphlogistischer und/oder antiandrogener Wirkung. Pflanzliche Extrakte die zur Auswahl stehen sind Extrakte aus Kürbiskernen *(Curcubita pepo),* Brennnesselwurzel *(Urtica dioica radix),* Zwergpalmenfrucht *(Serenoa repens, Sabal),* Roggenpollenextrakt *(Secale cereale)* und der afrikanischen Wurzelknolle *(Hypoxis rooperi*), Rotweinextrakt *(Vitis vinifera)* und Grapefruitkernextrakte *(Citrus (grandis) paradisi)* Rotweinextrakte sind unter anderem reich an Resveratrol (3,4',5-Trihydroxytransstilbene), einem Polyphenol mit antioxidativer Wirkung. Resveratol hat einen positiven Effekt auf artheriosklerotische Erkrankungen. In Zellkultur wurde auch ein antiproliferativer und proapoptotischer Effects auf die Androgen Rezeptor (AR)-negative Prostata Krebszelllinie, PC3 nachgewiesen (Sala et al., Drugs under experimental and clinical research, 29:263-9 (2003)). Weitere aktive Substanzen sind Acutissimin A, ein sogenanntes Flavano-Ellagitannin mit einem Flavonoid wie auch Tannin-Anteil (Angewandte Chemie Presseinformation Nr. 48/2003; Angewandte Chemie 115: 6194-6196 (2003)). Grapefruitkernextrakte sind Extrakte aus den Fruchtkernen sowie aus dem Fruchtmark (Teil des weißen Fruchtfleisches). Hier sind nützliche Inhaltsstoffe enthalten, die das genießbare Fruchtfleisch nicht bietet, insbesondere verschiedene antioxidativ-wirkende Polyphenole, wie Apigenin, Hesperidin, Naringin und Quercetin (Giamperi L. et al. Fitoterapia 75: 221-4 (2004)). Im Kernextrakt sind außerdem auch Terpene wie die Limonoide enthalten, die antikarzinogene Wirkung aufweisen (Jens Meyer-Wegener, Grapefruitkern-Extrakt - Das biologische Wundermittel, Mosaik-Verlag, 1997). In Kombination mit den Antioxidanzien enthalten im Traubenkernextrakt, dem Lycopin, sowie dem potenzstärkenden pflanzlichen Substanzen oder Extrakten, prosexuellen Extrakten, den Fettstoffwechsel-stimulierenden Extrakten haben sich diese Pflanzenextrakte als besonders wirkungsvoll erwiesen auf Verringerung von bereits existierenden Beschwerden verbunden mit der gutartigen Prostatavergrößerung (benigne Prostatasyndrom), bzw. der hinaus Verzögerung des Auftretens solcher Beschwerden. Bevorzugt ist hierbei eine Zusammensetzung, die Traubenkernextrakt *(Vitis vinifera),* Lycopin, Extrakte aus Muira puama *(Ptychopetalum olacoides Benth.; Ptychopetalum Anselmino),* Haferstroh *(Avena sativa),* Artischocke *(Cyanara cardunculus)* und Kürbiskernextrakt *(Curcubita pepo)* umfasst. Das Extrakt aus Kürbissamen vermindert den Harndrang, hemmt die wachstumsfördernde Testosteron Metaboliten und kräftigt die Muskulatur der Harnblase. Diese Zusammensetzung stärkt und kräftigt die Blasenfunktion, neben den oben bereits diskutierten anderen positiven Gesundheitseffekten.

Die Zusammensetzung kann weiter auf die männlichen Gesundheitsbelange hin optimiert werden, durch Zusatz von wenigstem einen pflanzlichen Extrakt mit blasentreibender Wirkung. Solch eine Zusammensetzung enthält vorzugsweise demnach wenigstens ein Extrakt, das ausgewählt ist aus Selleriewurzelextrakt *(Apium graveolens),* Preisselbeerextrakte *(Vaccinium vitis-idaea),* großfrüchtigen Moosbeerenextrakt ("Cranberries") *(Vaccinium macrocarpon)* und Wiesenküchenschellextrakt *(Pulsatilla pratensis).* Besonders bevorzugt ist hier Sellerieextrakt, da es neben der austreibenden und ausleitenden Wirkung auch entgiftend (entschlackend) und kräftigend wirkt. Eine Zusammensetzung umfassend Traubenkernextrakt *(Vitis vinifera),* Lycopin, Extrakten aus Muira puama *(Ptychopetalum olacoides Benth.; Ptychopetalum Anseimino),* Haferstroh *(Avena sativa),* Artischocke *(Cyanara cardunculus),* Kürbiskernen *(Curcubita pepo)* und Selleriewurzel *(Apium graveolens)* hat sich als besonders wirkungsvoll bezüglich dem allgemeinen Wohlbefinden und der Verbesserung der Gesundheit bei Männern erwiesen.

Die Vitalisierung des Mannes lässt sich weiter optimieren durch Zusatz von Mineralstoffen und/oder Spurenelementen zu den oben beschriebenen Zusammensetzungen. Bevorzugt sind hier Mineralstoffe und Spurenelemente, die bekanntermaßen als Antioxidanzen wirken, insbesondere Selen und Zink. Selen ist essentiell für den Schilddrüsenstoffwechsel, und die bei Selenmangel beobachtbare Müdigkeit beruht auf einer nicht optimalen Funktion der Schilddrüse. Selen ist weiterhin an der Radikalentgiftung beteiligt (Antioxidans) und dient somit auch dem molekularen Zellschutz, d. h. schützt insbesondere Proteine und Nukleinsäuren vor oxidativen Veränderungen. Zink unterstützt Zellteilungsprozesse, fördert den Aufbau von Bindegewebe, und ist wichtig für das Immunsystem und Spermatogenese. Daher ist besonders bevorzugt eine Zusammensetzung umfassend Traubenkernextrakt *(Vitis vinifera),* Lycopin, Extrakte aus Muira puama *(Ptychopetalum olacoides Benth.; Ptychopetalum Anselmino),* Haferstroh *(Avena sativa),* Artischocke *(Cyanara cardunculus),* Kürbiskernen *(Curcubita pepo)* und Selleriewurzel *(Apium graveolens),* sowie mit Selen und Zink. Bei subfertilen Männern, wurde durch Gabe von Zink eine Verbesserung der Spermienqualität (WHO Standard) beobachtet (Wong *et al.,* Fertility and Sterility 77(3): 491-8 (2002)). Übereinstimmend mit dieser Beobachtung hat die erfindungsgemäße Zusammensetzung umfassend Traubenkernextrakt *(Vitis vinifera),* Lycopin, Extrakte aus Muira puama *(Ptychopetalum olacoides Benth.; Ptychopetalum Anselmino),* Haferstroh *(Avena sativa),* Artischocke *(Cyanara cardunculus),* Kürbiskernen *(Curcubita pepo)* und Selleriewurzel *(Apium graveolens),* sowie Selen und Zink nicht nur das allgemeine Wohlbefinden von Männern verbessert, und die Infektanfälligkeit reduziert (gestärktes Immunsystem), sondern auch die Spermienqualität (Beurteilung nach WHO Standard: Konzentration, Motilität und Morphologie) verbessert.

Die Zusammensetzung konnte weiter optimiert werden durch den Einschluss von für das männliche Wohlbefinden geeigneten Vitaminen. Solche Vitamine sind z. B. Folsäure, Biotin, Vitamin C, Vitamin B6 und Carotine, die keine ψψ-Carotine sind, insbesondere ß-Carotin. Folsäure ist wesentlich beteiligt am Eiweißstoffwechsel (Transportfunktion), an der Blutbildung, und vermindert Herz-Kreislauf-Erkrankungen. Biotin spielt ebenfalls eine wichtige Rolle bei Stoffwechselprozessen, es fördert das Zellwachstum und den Abbau von Amino-und Fettsäuren. Vitamin C ist eine Antioxidans, und regt auch die Bildung von Kollagen in Haut, Bindegewebe, Knochen und Knorpel an. Vitamin E ist ebenfalls eine Antioxidans, ist beteiligt an der Blutbildung, wirkt entzündungshemmend, und eignet sich zur Arthroseprophylaxe. Als besonders wirksam hat sich dabei eine Zusammensetzung umfassend Traubenkernextrakt *(Vitis vinifera),* Lycopin, Extrakte aus Muira puama *(Ptychopetalum olacoides Benth.; Ptychopetalum Anselmino),* Haferstroh *(Avena sativa),* Artischocke *(Cyanara cardunculus),* Kürbiskernen *(Curcubita pepo)* und Selleriewurzel *(Apium graveolens),* Selen, Zink, Folsäure, Biotin, Vitamin C, Vitamin B6 und ß-Carotin erwiesen. Diese Kombination hat sich als optimal erwiesen, da die ausgewählten Vitamine die anderen Bestandteile in ihrer Wirkung komplementieren bzw. verstärken, d. h. Synergieeffekte sich einstellen. Die Mineralstoffe und Vitamine wurden als ein Premix der Zusammensetzung beigefügt.

Die Mengenverhältnisse der Einzelbestandteile wurden optimiert, eine besonders bevorzugte Zusammensetzung ist in nachstehender Tabelle in Gewichtsprozent (Gew.-%) zusammengefasst (Angabe).

**Tabelle 1:**

| Bevorzugte Zusammensetzungen | |
|---|---|
| Bestandteil | Gew.-% |
| Traubenkern-Extrakt | 4,90 - 14,76 |
| Lycopin | 0,90 - 2,7 |
| Muara-puama-Extrakt | 0,90-2,7 |
| Haferstroh-Extrakt | 14,51 - 43,53 |
| Artischocken-Extrakt | 3,92- 11,76 |
| Kürbiskern-Extrakt | 13,73 - 41,18 |
| Selleriewurzel-Extrakt | 2,72-8,16 |
| Premix* | 29,41 - 88,24 |

| | |
|---|---|
| * Gemäß der Mengen an Vitaminen und Mineralstoffen im Premix, liegen bei den gewählten Gew.-% des Premixes zwischen 10 - 30 µg Selen, 1 - 5 mg Zink, 100 - 300 µg Folsäure, 33 - 100 µg Biotin, 30 - 100 mg Vitamin C, 3,7 - 14 mg Vitamin E, 0.5 -1,5 mg Vitamin B6 und 1 - 3 mg ß-Carotin vor. | |

Erfindungsgegenstand ist ebenfalls eine der oben beschriebenen Zusammensetzungen in Form einer Kapsel oder einer Tablette. Somit ist auch eine Kapsel, die die erfindungsgemäße Zusammensetzung enthält, ein Erfindungsgegenstand. Kapseln im Sinne der Erfindung sind Cellulose- (Hydroxypropylmethylcellulose (HPMC)), Weichgelatine- oder Hartgelatinekapseln. Ebenso sind magensaftresistente Kapseln erfindungsgemäß. Erfindungsgemäß ist auch ein Verfahren zur Herstellung eines Nahrungsergänzungsmittels umfassend eine der oben beschriebenen Zusammensetzungen geeignet zur Prävention von männerspezifischen Alterungserscheinungen, insbesondere sexueller Dysfunktion, Herz-Kreislauf Problemen, Fettstoffwechselerkrankungen und Blasenschwäche. Die erfindungsgemäße Zusammensetzung wirkt verschiedenen Mangelerscheinungen bei Männern entgegen, die allgemein als natürliche Alterungserscheinungen abgetan werden. Die erfindungsgemäße Zusammensetzung wirkt gegen Erschöpfung, Lustlosigkeit und Impotenz durch das Muira puama Extrakt, wirkt gegen Beschwerden beim Wasserlassen (benignes Prostatasyndrom, Prostatahyperplasie) durch das Kürbiskernextrakt, wirkt gegen Leberschwäche, Störungen des Gallenfluss und erhöhten Cholesterinspiegel durch das Artischockenextrakt, wirkt gegen Erschöpfung und Lustlosigkeit und Blasenschwäche durch Selleriewurzel, wirkt gegen Schwächezustände, Erschöpfung und Lustlosigkeit durch das Haferstrohextrakt, wirkt dem Prostatakrebsrisiko entgegen durch Lycopin, vermindert das Krebsrisiko und Artherioskleroserisiko durch verschiedene Antioxidanzien oder Extrakten reich an Antioxidanzien, wie Traubenkernextrakt, Selen, Zink, Vitamin C und Vitamin E, wirkt gegen Haarausfall und brüchige Nägel durch Biotin, wirkt gegen reduzierte Fruchtbarkeit und Infektanfälligkeit durch Zink, wirkt gegen Müdigkeit, Abgespanntheit und Infektanfälligkeit durch Vitamin C und wirkt gegen Nervosität und Müdigkeit durch Vitamin B6.

Die Erfindung wird anhand der nachfolgenden Beispielen näher erläutert, die jedoch den Schutzbereich nicht einschränken.

### Beispiele

### Materialien und Methoden

Die Formulierung wurde von einem Lohnhersteller, der Firma Klocke Pharma Service GmbH (NL 77767 Appenweier) durchgeführt. Die Klocke Pharma Service GmbH hat die einzelnen Rohstoffe von diversen Händlern bezogen.
Bei dem Traubenkernextrakt (Finzelberg GmbH & Co. KG; Chargen-Nr. 03121083) wurde Wasser gemäß der Finzelberg-Monographie als Auszugsmittel verwendet. Das Wasser genügte den in Ph. Eur. Suppl. 2000 gestellten Anforderung bezüglich der Eigenschaften und Reinheit. Das Verhältnis von der Zubereitung (= nativer Extrakt bzw. Wirkstoff) ist durchschnittlich 8-12:1. Bei dem Traubenkernextrakt handelt es sich um ein Trockenextrakt. Der Wassergehalt (%) wurde gemäß des europäischen Arzneibuchs (Ph. Eur.) nach der Karl-Fischer-Methode bestimmt und betrug 4.1 %. Die Karl-Fischer Methode wird in der Lebensmittelanalytik als amtliche Methode angewendet (§ 35 LMBG) Die Korngröße, d. h. der Anteil der Partikel die kleiner als 100 µm sind betrug 94,8 %. Die Schütteldichte in g/l analog zu DIN 53912 betrug 516 g/l. Polyphenole (Procyanidin) machen 33,5 % der Trockenmasse aus.

Als Lycopin (3 % CWD) wurde in kaltem Wasser dispergierendes natürliches, aus Tomaten gewonnenes Lycopin verwendet, das auf einer Matrix aus Maltodextrin sprühgetrocknet wurde. Die Tomaten waren reif und zeichneten sich durch einen besonders hohen Lycopin-Anteil aus. Nach spektrophotometrischen Analysen beträgt der Lycopin-Anteil an dem sprühgetrockneten Pulver 3 %, nach einem spezifischen Lycopin Assay 2,5 %.

Das Muira-puama-Extrakt wurde aus dem Holz der Stammpflanzen *Ptychopetalum olacoides Benth.* und *Ptychopetalum Anselmino* gewonnen. Als Auszugsmittel wurde 70 Vol.-% Ethanol verwendet. Das Verhältnis von Wirkstoff zu Extraktzubereitung beträgt 10:1, das Verhältnis von Droge zu Drogenzubereitung (= nativer Extrakt) 75:1 (50 -100:1). Das Extrakt setzt sich zusammen aus 10 - 20 % nativer Extraktanteil (Muira puama), 58,5 - 66 % Lactose-Monohydrat (Ph. Eur.), 19,5 - 22 % Cellulosepulver (Ph. Eur.) und maximal 2 % hochdispergierendes SiO₂ (Ph. Eur.). Die Identitätsprüfung erfolgte mittels Dünnschichtchromatographie. Die Teilchengröße beträgt maximal 1 mm (Ph. Eur.), und die Schütteldichte 500 - 750 g/l (Ph. Eur.).

Das Haferstrohextrakt wurde von der Stammpflanze *Avena sativa* gewonnen. Der Drogenanteil wird von der grünen Frischpflanze gewonnen. Als Auszugsmittel wurde Aqua purificata verwendet. Das Droge Extrakt Verhältnis (DEV) nativ beträgt 12,0-18,0 = 1 (15,0 = 1, bezogen auf die Frischpflanze), und das Droge Extrakt Verhältnis (DEV) zubereitet beträgt 8,0 - 12,0 = 1 (10,0 = 1, bezogen auf die Frischpflanze). Das Droge Extrakt Verhältnis (DEV) nativ bezogen auf die trockene Droge beträgt 4,0-6,0 = 1. Die Identität des Extraktes wurde mit der Flachsmann Methode bestimmt. Die Schütteldichte beträgt 300 - 600 g/l. Als Trägerstoff wurde Maltodextrin eingesetzt.

Das Artischockenextrakt wurde aus den Blättern der Stammpflanze *Cynara scolymus* gewonnen. Als Auszugsmittel wurde Aqua purificata verwendet. Das Droge Extrakt Verhältnis (DEV) nativ beträgt 22,0-28,5 = 1 (25 = 1, bezogen auf die frische Droge), und das Droge Extrakt Verhältnis (DEV) zubereitet beträgt 13,2 - 17,1 = 1 (15,0 = 1, bezogen auf die frische Droge). Das Droge Extrakt Verhältnis (DEV) nativ bezogen auf die trockene Droge beträgt 5,8-7,5 = 1, und das Droge Extrakt Verhältnis (DEV)zubereitet bezogen auf die trockene Droge beträgt 3,5-4,5 = 1. Die Identität des Extraktes wurde mit der Flachsmann Methode bestimmt. Die Schütteldichte beträgt 300 - 600 g/l. Als Trägerstoff wurde Maltodextrin eingesetzt (40 %). An Cynarin Derivaten enthält das Extrakt mehr als 2 %.

Das Kürbiskernextrakt wurde aus frischen Kürbissamen hergesteht. Als Auszugsmittel wurde 60 Massen % Ethanolum verwendet. Das Droge Extrakt Verhältnis (DEV) nativ beträgt 26,0-31,0 = 1 (28,5 = 1), und das Droge Extrakt Verhältnis (DEV) zubereitet beträgt 6,5 - 7,15 = 1. Als Trägerstoff wurde Lactosum monohydricum verwendet. Die Schütteldichte beträgt 300 - 600g/l. Das Selleriewurzelextrakt wurde aus der Wurzel der Stammpflanze *Apium graveolens* gewonnen. Als Auszugsmittel wurde 70 Vol.-% Ethanol verwendet. Das Verhältnis von Droge zu Drogenzubereitung (natives Extrakt) beträgt 3,5 - 5,5 : 1 konzentriert. Das Extrakt setzt sich zusammen aus 80 % nativen Extraktanteil und 20 % Maltodextrin als Trägerstoff. Die Teilchengröße beträgt maximal 1 mm, und die Schütteldichte 500 - 800 g/l.

Für die Vitamine und Mineralien wurde ein Premix verwendet, der die in der Tabelle dargestellte Zusammensetzung hat. Eine Dosiseinheit sind 300 mg, und Trägerstoff ist Maltodextrin. Bei der nachfolgenden Formulierung der Zusammensetzung wurde 1/3 der Dosiseinheit, d. h. 100 mg des Premixes verwendet.

**Tabelle 2:**

| Premix-Zusammensetzung | | | |
|---|---|---|---|
| Komponente | Gehalt pro Dosiseinheit | Gehalt pro kg Mischung | Qualität der eingesetzten Rohstoffe |
| β-Carotin | 3,0 mg | 12000 mg | B-Carotin, 20 % granuliert, Lebensmittelqualität |
| Vitamin B6 | 1,5 mg | 6250 mg | Pyridoxin-Hydrochlorid, EP |
| Vitamin C | 100.0 mg | 416667 mg | Ascorbinsäure, EP |
| Vitamin E | 14,0 mg | 53667 mg | D,L-alpha-Tocopherylacetat, CWD, gelatinefrei (EP), Pulver, 50 %, Matrix aus modifizierter Stärke und Gummi arabicum, Lebensmittelqualität |
| Biotin | 100,0 µg | 450000 µg | Biotin, EP |
| Folsäure | 300,0 µg | 3000000 µg | Pteroylglutaminsäure, EP |
| Zink | 5,0 mg | 16667 mg | Zinkgluconat, Monohydrat, USP |
| Selen | 30,0 µg | 100000 µg | Natriumselenit, 1 % auf Maltodextrin, Lebensmittelqualität |

### Beispiel 1: Formulierung der Zusammensetzung

Tabelle 3 zeigt die verwendete Rezeptur. Spalte 1 der Tabelle zeigt die Rohstoffbezeichnung, Spalte 2, die jeweilig verwendete Menge in mg bzw. in µg, Spalte 3 zeigt den prozentualen Anteil des jeweiligen Rohstoffs. In Spalte 4 sind die 1,75-fachen Mengen eines jeden Rohstoffs aufgeführt, wie sie verwendet wurden, um ein Ansatzgemisch zusammenzustellen, aus dem dann die Einzeldosis, nämlich 340 mg abgewogen wurde. Die 340 mg Einzeldosis stellt die Kapselfüllmenge dar, d. h. die Menge die in eine gelatinefreie Cellulose Kapsel (Hydroxypropylmethylcellulose (HPMC) Kapsel, eingefüllt wurde. Zur Vereinfachung wurde in den unteren Teil der Tabelle nochmals die Einzelkomponenten des Premixes aufgeführt (siehe auch Tabelle 2), und in welchen Mengen sie in der Einzeldosis vorliegen.

**Tabelle 3**

| Rohstoff | Einzeldosis | %-Anteil | 1,75-facher Ansatz |
|---|---|---|---|
| Lycopin 3 % CWD | 3,056 mg | 0,899 % | 5,348 mg |
| Traubenkern TE, 8-12:1 (natives Extra kt) | 16,667 mg | 4,902 % | 152,952 mg |
| Muira-puama-Holz TE; 10:1 (Droge: Drogenzubereitung) | 10,000 mg | 2,941 % | 17,500 mg |
| Haferstroh TE, 10:1, 65 % nativ | 49,333 mg | 14,510 % | 86,333 mg |
| Artischocken TE, 15:1, 60 % nativ | 13,333 mg | 3,921 % | 23,333 mg |
| Kürbissamen TE, 7,15:1; 35 % nativ | 46,667 mg | 13,726 % | 81,667 mg |
| Selleriewurzel TE, 3,5-5,5:1 | 9,260 mg | 2,724 % | 16,205 mg |
| Premix (siehe *) | 100,000 mg | 29,412 % | 175,000 mg |
| Lactose, EuAB | 87,401 mg | 25,706 % | 152,952 mg |
| Siliciumdioxid, EuAB, gefällt | 1,716 mg | 0,505 % | 3,003 mg |
| Magnesiumstearat, EuAB, pflanzlich | 2,567 mg | 0,755 % | 4,492 mg |
| Mischung gesamt | 340,00 mg | 100,00 % | 595,00 mg |
| | | | |
| * Premix (Trägerstoff: Maltodextrin) | | | |
| β-Carotin | 1,000 mg | | |
| Vitamin B6 | 0,500 mg | | |
| Vitamin C | 33,333 mg | | |
| Vitamin E | 4,666 mg | | |
| Biotin | 33,333 µg | | |
| Folsäure | 100,00 µg | | |
| Zink | 1,666 mg | | |

Diese Rezeptur enthält neben den aktiven pflanzlichen Drogenbestandteilen und den Vitaminen und Mineralstoffen auch Trägerstoffe. Bei den Trägerstoffen handelt es sich um Magnesiumstearat, Siliciumdioxid und Lactose.

## Patentansprüche

1. Zusammensetzung umfassend
(a) eine polyphenolhaltige Substanz pflanzlichen Ursprungs, (b) eine ψψ-Carotinverbindung und (c) wenigstens eine potenzstärkende und/oder prosexuelle Substanz.

2. Die Zusammensetzung gemäß Anspruch 1, wobei
(i) die polyphenolhaltige Substanz ein Extrakt oder Öl von Traubenkernen, Apfelkernen, Grapefruitkernen oder Rotwein ist, und vorzugsweise Traubenkernextrakt ist; und/oder
(ii) die ψ,ψ-Carotinverbindung ein ψ,ψ-Carotin ist, und vorzugsweise Lycopin ist; und/oder
(iii) die potenzstärkende Substanz ein Extrakt vorzugsweise ausgewählt aus Muira-puama-Holz *(Ptychopetalum olacoides Benth.; Ptychopetalum Anselmino),* Yohimbe-Rinde *(Corynanthe yohimbe),* Damiana *(Turnera diffusa),* Maca-Wurzel *(Maca andina; Lepidium meyenii),* Sibirischer Ginseng (Eleutherococcus senticosus maxim (Acanthopanax senticosus)) Ginseng (Panax ginseng), Catuaba-Rinde *(Erythroxylon catuaba),* und Cola-Nuss *(Coala vera)* ist; und/oder
(iv) die prosexuelle Substanz ein pflanzliches Extrakt, vorzugsweise ausgewählt aus Haferstroh (Avena sativa) ist.

3. Zusammensetzung nach Anspruch 1 oder 2 wobei das Gewichtsverhältnis (a) : (b): von 10 : 1 bis 0,4 : 1 und/oder (a):(c) von 0,03 : 1 bis 25 : 1 beträgt.

4. Zusammensetzung gemäß einem der Ansprüche 1 bis 3, weiterhin umfassend
(d) wenigstens eine pflanzliche Substanz, die den Fettstoffwechsel stimuliert, insbesondere ein Fettstoffwechsel-stimulierendes Extrakt oder Öl, das besonders bevorzugt ausgewählt ist aus Artischockenblättern *(Cyanara cardunculus),* Löwenzahn *(Taraxacum officinale),* Mariendistel *(Carduus marianus),* Schwarzkümmel, Bockshornklee *(Trigonella foenum-graecum)* und dem Harz des Guggul-Baums *(Commiphora mukul)*
(e) eine pflanzliche Substanz mit antikongestiver, antiproliferativer, antiphlogistischer und/oder antiandrogener Wirkung auf das benigne Prostatasyndrom, insbesondere ein Extrakt, das ausgewählt ist aus Kürbiskern *(Curcubita pepo),* Brennesselwurzel *(Urtica dioica radix),* Zwergpalmenfrucht *(Serenoa repens, Sabal),* afrikanische Wurzelknolle *(Hypoxis rooperi),* Grapefruitbestandteile und Roggenpollenextrakt (*Secale cereale)* und Rotweinextrakt, ist; und/oder
(f) eine Substanz mit harntreibender Wirkung insbesondere ein Extrakt ausgewählt aus Selleriewurzel *(Apium graveolens),* Preisselbeere *(Vaccinium vitis-idaea),* großfrüchtigen Moosbeere (Cranberries), *(Vaccinium macrocarpon)* und Wiesenküchenschellextrakt (Pulsatilla pratensis) ist; und/oder
(g) Mineralstoffe und/oder Spurenelemente, insbesondere ausgewählt aus Selen, Zink; und/oder
(h) Vitamine, insbesondere Folsäure, Biotin, Vitamin C, Vitamin E, Vitamin B6 und Carotinverbindung, die keine ψ,ψ-Carotinverbindung ist, besonders bevorzugt ß-Carotin.

5. Zusammensetzung nach Anspruch 4, wobei die Zusammensetzung 2-25, vorzugsweise 4-15 Gew.-% (a), 0.3-5, vorzugsweise 0,7-30 Gew.-% (b), 1-60, vorzugsweise 2-50 Gew.-% (c), 5-60, insbesondere 10-50 Gew.-% (e), 1-15, vorzugsweise 2-10 Gew.-% (f), und 5-35, vorzugsweise 10-20 Gew.-% eines Prämixes aus (g) und (h) enthält.

6. Zusammensetzung gemäß einem oder mehreren der Ansprüche 1 bis 5, umfassend Muira-puama-Holz Extrakt, Traubenkernextrakt, Lycopin, Haferstrohextrakt, Artischockenextrakt, Kürbiskernextrakt und Selleriewurzelextra kt.

7. Zusammensetzung gemäß Anspruch 6, umfassend 0,8 - 2,7 Gew.-% Muira-puama-Extrakt, 4,9 - 14,8 Gew.-% Traubenkernextrakt, 0,8 - 2,7 Gew.-% Lycopin, 14 - 44 Gew.-% Haferstroh Extrakt, 3,9 - 11,8 Gew.-% Artischockenblätter Extrakt, 13 - 41 Gew.-% Kürbiskern Extrakt, 2,7 - 8,2 Gew.-% Selleriewurzel Extrakt,
und ein Spurenelement-Vitamin Premix enthaltend 10 - 30 µg Selen, 1-5 mg Zink, 100 - 300 µg Folsäure, 33 - 100 µg Biotin, 30 - 100 mg Vitamin C, 3,7 - 14 mg Vitamin E, 0.5 -1,5 mg Vitamin B6 und 1 - 3 mg ß-Carotin.

8. Zusammensetzung gemäß Ansprüchen 6 oder 7, umfassend 10 mg Muira-puama-Extrakt, 16 mg Traubenkernextrakt, 3 mg Lycopin, 49 mg Haferstroh Extrakt, 13 mg Artischocken Extrakt, 46 mg Kürbiskern Extrakt, 9 mg Selleriewurzel Extrakt, 30 µg Selen, 1 mg Zink, 100 µg Folsäure, 33 µg Biotin, 30 mg Vitamin C, 3,7 mg Vitamin E, 0.5 mg Vitamin B6, 1 mg ß-Carotin.

9. Zusammensetzung gemäß einem oder mehren der Ansprüche 1 bis 8, das ein Nahrungsergänzungsmittel oder Arzneimittel zur Prävention und/oder Behandlung von männerspezifischen Alterungserscheinungen, insbesondere ausgewählt aus der Gruppe von sexueller Dysfunktion, Herz-Kreislauf Problemen, Fettstoffwechselerkrankungen und Blasenschwäche ist.

10. Zusammensetzung gemäß einem oder mehreren der Ansprüche 1 bis 9, die in Form einer Kapsel oder einer Tablette, insbesondere einer Cellulose-, Weichgelatine- oder Hartgelatinekapsel besonders bevorzugt einer magensaftresistente Kapsel vorliegt.

11. Ein Verfahren zur Herstellung einer Zusammensetzungen gemäß Ansprüchen 1 bis 10 umfassend das Vermischen der Komponenten (a) bis (h).

12. Kapsel oder Tablette enthaltend eine Zusammensetzung gemäß einem oder mehreren der Ansprüche 1 bis 9.
